# EUROPEAN PATENT APPLICATION

(11) **EP 3 103 381 A1**
(43) Date of publication of application: **14.12.2016**
(21) Application number: 15853210.1
(22) Date of filing: 18.08.2015
(51) Int. Cl.: A61B 1/04, G02B 23/24, H04N 5/225

(54) **SOLID-STATE IMAGING DEVICE AND ELECTRONIC ENDOSCOPE PROVIDED WITH SOLID-STATE IMAGING DEVICE**

(30) Priority: 20.10.2014 JP 2014213605
(71) Applicant: Olympus Corporation, Tokyo 192-8507 (JP)
(72) Inventor: ICHIMURA Hironobu, Hachioji-shi Tokyo 192-8507 (JP); TAKAHASHI Tomohisa, Hachioji-shi Tokyo 192-8507 (JP); KIMURA Hiroyuki, Hachioji-shi Tokyo 192-8507 (JP)
(74) Representative: Gunzelmann, Rainer
(86) International application number: PCT/JP2015/073112
(87) International publication number: WO 2016/063603

(57) **Abstract**

A solid-state image pickup apparatus 1 includes an image pickup device 4 configured to detect an object image, a rigid board 5 electrically connected to the image pickup device 4, and a signal cable 115 incorporating at least one or more coaxial lines 42. The rigid board 5 includes a first surface including a core-wire connecting section 5a to which core wires 42a of the coaxial lines 42 are connected and a second surface including an external-conductor connecting section 5b to which external conductors 42c of the coaxial lines 42 are connected, the second surface being substantially parallel to the first surface. The coaxial lines 42 are connected substantially in parallel to the first and second surfaces of the rigid board 5.

## Description

### Technical Field

The present invention relates to a solid-state image pickup apparatus disposed at a distal end portion of an insertion section of an electronic endoscope.

### Background Art

In order to observe a part where observation is difficult such as an inside of a body of an organism or an inside of a structure, an electronic endoscope that can be led from an outside into the inside of the organism or the structure and includes an image pickup unit, which is a solid-state image pickup apparatus, for picking up an optical image has been used in, for example, a medical field or an industrial field.

The image pickup unit of the electronic endoscope includes an objective lens that forms an object image and an image pickup device, which is generally a CCD (charge coupled device) a CMOS (complementary metal oxide semiconductor) sensor, or the like, disposed on an image forming surface of the objective lens.

As such an electronic endoscope, for example, an image pickup unit disclosed in Japanese Patent Application Laid-Open Publication No. 2005-304876 is known. The conventional image pickup unit of the electronic endoscope has a configuration in which external conductors of a plurality of coaxial signal lines are wound around a jumper wire for ground and connected to a circuit board.

Incidentally, in an electronic endoscope in recent years, for improvement of insertability into a subject, a reduction in a diameter and a reduction in length of an insertion section are requested. Accordingly, a reduction in size of an image pickup unit is also requested.

However, in the conventional image pickup unit, a shield bundle obtained by binding shields of the plurality of coaxial signal lines is electrically connected to the circuit board via the jumper wire for ground. Therefore, there is a problem that not only a reduction in length in a longitudinal direction of a laminated board is hindered but also a reduction in a diameter is hindered because a bound section of the external conductors bulges in an outer diameter direction.

When the reduction in the size of the image pickup unit is hindered in this way, a distal end portion of the insertion section in which the image pickup unit is incorporated increases in length and an outer diameter of the insertion section increases. This not only hinders the reduction in the diameter of the insertion section but also deteriorates insertability because a rigid length increases.

Therefore, the present invention has been devised in view of the circumstances and an object of the present invention is to provide a small solid-state image pickup apparatus contributing to a reduction in a diameter of an insertion section and a reduction in a rigid length and an electronic endoscope including the solid-state image pickup apparatus.

### Disclosure of Invention

### Means for Solving the Problem

A solid-state image pickup apparatus according to an aspect of the present invention is a solid-state image pickup apparatus including: an image pickup device configured to detect an object image; a rigid board electrically connected to the image pickup device; and a signal cable incorporating at least one or more coaxial lines. The rigid board includes: a first surface including a core-wire connecting section to which core wires of the coaxial lines are connected; and a second surface including an external-conductor connecting section to which external conductors of the coaxial lines are connected, the second surface being substantially parallel to the first surface. The coaxial lines are connected substantially in parallel to the first and second surfaces of the rigid board.

An electronic endoscope according to another aspect of the present invention includes: a solid-state image pickup apparatus including: an image pickup device configured to detect an object image; a rigid board electrically connected to the image pickup device; and a signal cable incorporating at least one or more coaxial lines, the rigid board including: a first surface including a core-wire connecting section to which core wires of the coaxial lines are connected; and a second surface including an external-conductor connecting section to which external conductors of the coaxial lines are connected, the second surface being substantially parallel to the first surface, and the coaxial lines being connected substantially in parallel to the first and second surfaces of the rigid board; and an insertion section, in a distal end portion of which the solid-state image pickup apparatus is incorporated. The signal cable is disposed substantially in parallel to a longitudinal direction of the insertion section.

According to the present invention described above, it is possible to provide a small solid-state image pickup apparatus contributing to a reduction in a diameter of a distal end of an insertion section and a reduction in a rigid length and an electronic endoscope including the solid-state image pickup apparatus.

### Brief Description of the Drawings

Fig. 1 is a diagram showing a configuration of an endoscope in an aspect of the present invention;
Fig. 2 is a front view showing a configuration of a distal end portion of an insertion section in the aspect of the present invention;
Fig. 3 is a sectional view showing the configuration of the distal end portion taken along line III-III in Fig. 2 in the aspect of the present invention;
Fig. 4 is a sectional view showing the configuration of the distal end portion taken along line IV-IV in Fig. 3 in the aspect of the present invention;
Fig. 5 is a partial sectional view showing a configuration of an image pickup unit in the aspect of the present invention;
Fig. 6 is a sectional view showing a configuration of a composite cable in the aspect of the present invention;
Fig. 7 is a sectional view showing a configuration of an image pickup unit taken along line VII-VII in Fig. 5 in the aspect of the present invention;
Fig. 8 is a sectional view showing the configuration of the image pickup unit taken along line VIII-VIII in Fig. 5 in the aspect of the present invention;
Fig. 9 is a partial sectional view showing a configuration of a circuit board to which a signal line is connected in the aspect of the present invention;
Fig. 10 is a sectional view showing a configuration of an external-conductor connecting section of a circuit board of an image pickup unit in a first modification of the aspect of the present invention;
Fig. 11 is a side view showing a configuration of a circuit board to which a power supply line and a plurality of signal lines are connected in a second modification of the aspect of the present invention;
Fig. 12 is a sectional view showing a configuration of an image pickup unit taken along line XII-XII in Fig. 11 in the aspect of the present invention;
Fig. 13 is a side view showing a configuration of a circuit board to which a power supply line and a plurality of signal lines are connected in a third modification of the aspect of the present invention;
Fig. 14 is a sectional view showing a configuration of an image pickup unit taken along line XIV-XIV in Fig. 13 in the aspect of the present invention;
Fig. 15 is a side view showing a state before an external-conductor connecting section is bonded to a core-wire connecting section in the aspect of the present invention;
Fig. 16 is a side view showing a configuration of a circuit board to which a plurality of signal lines are connected in a fourth modification of the aspect of the present invention;
Fig. 17 is a sectional view showing a configuration of an image pickup unit taken along line XVII-XVII in Fig. 16 in the aspect of the present invention;
Fig. 18 is a sectional view showing the configuration of the image pickup unit taken along line XVIII-XVIII in Fig. 16 in the aspect of the present invention;
Fig. 19 is a perspective view showing a configuration of an external-conductor connecting section made of metal in the aspect of the present invention;
Fig. 20 is a top view showing a configuration of a circuit board to which a plurality of signal lines are connected in a fifth modification of the aspect of the present invention;
Fig. 21 is a side view showing a configuration of a circuit board to which a plurality of signal lines are connected in the aspect of the present invention; and
Fig. 22 is a sectional view showing a configuration of an image pickup unit taken along line XXII-XXII in Fig. 21 in the aspect of the present invention.

### Best Mode for Carrying Out the Invention

A preferred mode of the present invention is explained below with reference to the drawings. Note that, in the respective figures referred to in the following explanation, scales are differentiated for each of components in order to show the respective components in sizes recognizable on the drawings. The present invention is not limited to only numbers of components, shapes of the components, ratios of sizes of the components, and relative positional relations of the respective components described in the figures. In the following explanation, in some case, upper and lower directions in view toward paper surfaces of the figures are explained as upper sections and lower sections of the components and an inner side direction of the components is explained as an external form center direction or the like.

First, an image pickup unit functioning as a solid-state image pickup apparatus and an electronic endoscope in an aspect of the present invention are explained below with reference to the drawings.

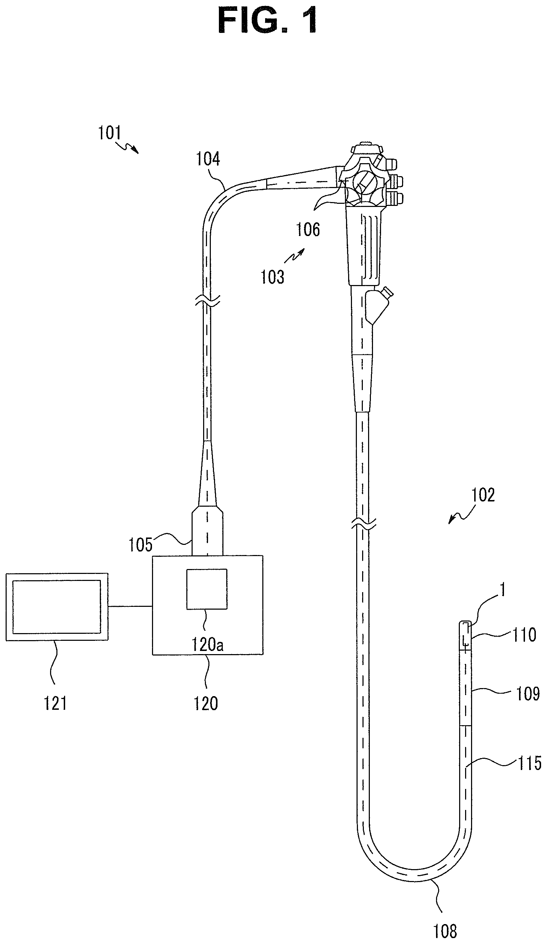

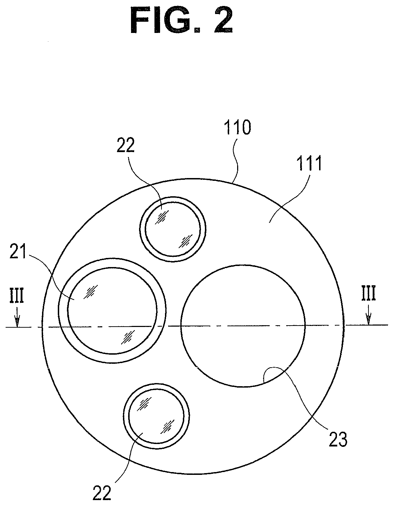

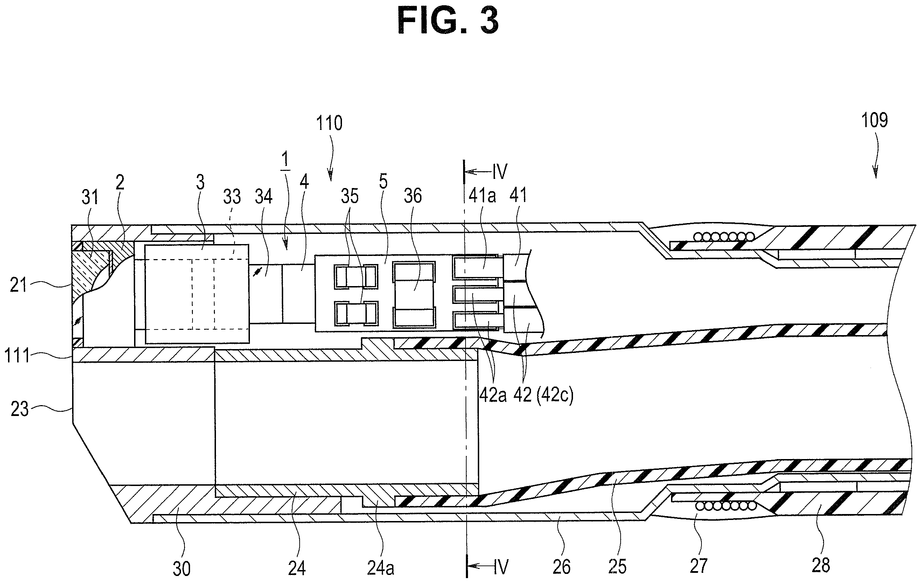

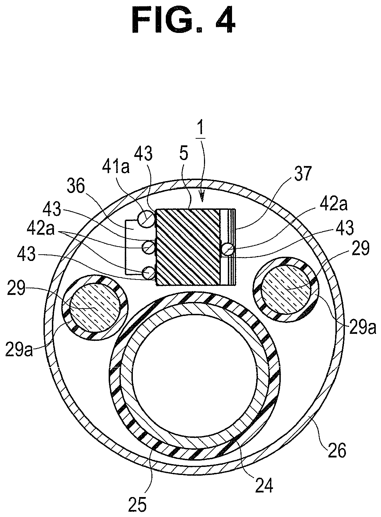

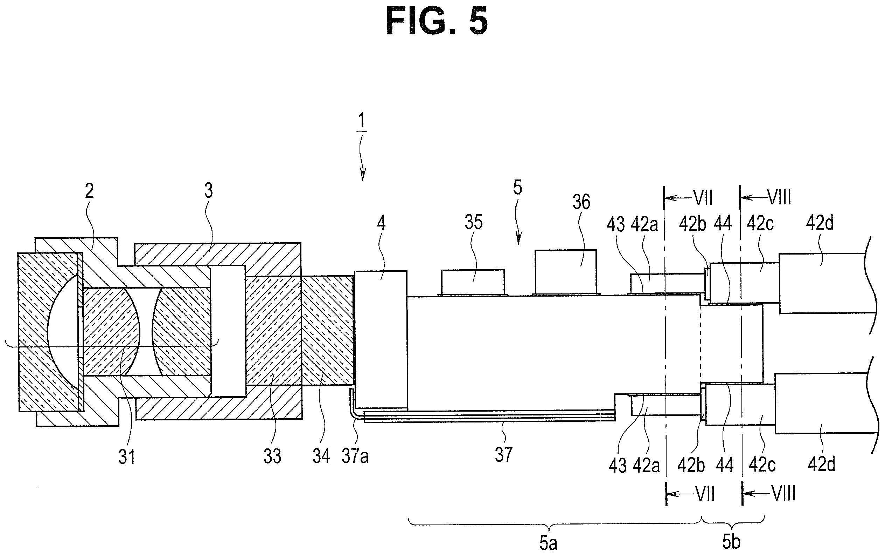

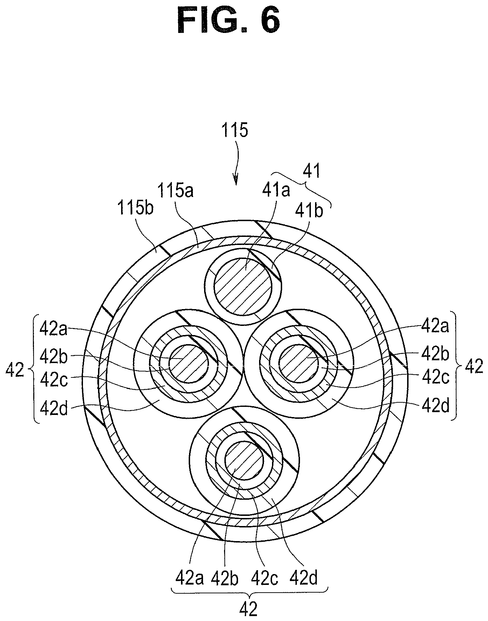

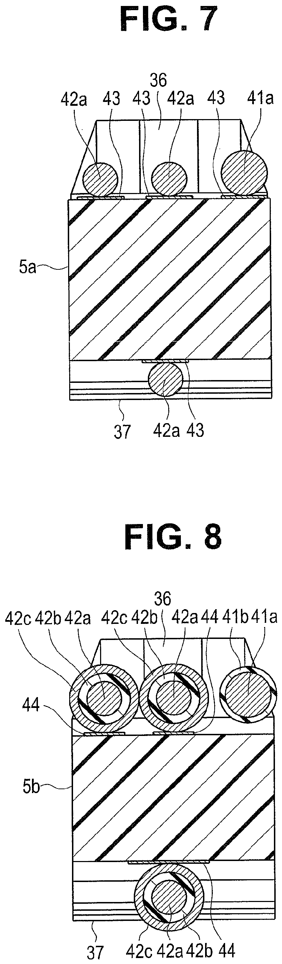

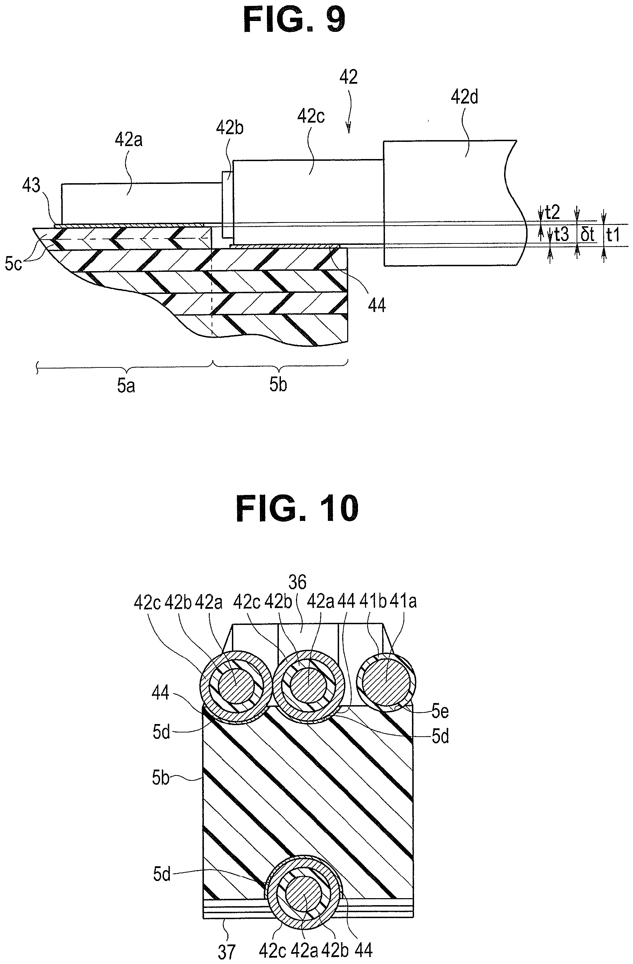

Note that Fig. 1 is a diagram showing a configuration of an endoscope, Fig. 2 is a front view showing a configuration of a distal end portion of an insertion section, Fig. 3 is a sectional view showing the configuration of the distal end portion taken along line III-III in Fig. 2, Fig. 4 is a sectional view showing the configuration of the distal end portion taken along line IV-IV in Fig. 3, Fig. 5 is a partial sectional view showing a configuration of an image pickup unit, Fig. 6 is a sectional view showing a configuration of a composite cable, Fig. 7 is a sectional view showing a configuration of an image pickup unit taken along line VII-VII in Fig. 5. Fig. 8 is a sectional view showing the configuration of the image pickup unit taken along line VIII-VIII in Fig. 5. Fig. 9 is a partial sectional view showing a configuration of a circuit board to which a signal line is connected.

First, an example of a configuration of an electronic endoscope (hereinafter simply described as endoscope) 101 including an image pickup unit 1 functioning as a solid-state image pickup apparatus according to the present invention is explained with reference to Fig. 1.

The endoscope 101 in the present embodiment has a configuration that is insertable into a subject such as a human body and optically picks up a predetermined observation part in the subject.

Note that the subject into which the endoscope 101 is led is not limited to the human body and may be another organism or may be an artificial object such as a machine or a building.

The endoscope 101 is mainly configured by an insertion section 102 led into an inside of the subject, an operation section 103 located at a proximal end of the insertion section 102, and a universal cord 104 extending from the operation section 103.

The insertion section 102 is configured by consecutively connecting a distal end portion 110 disposed at a distal end, a bendable bending section 109 disposed on a proximal end side of the distal end portion 110, and a flexible tube section 108 having flexibility disposed on a proximal end side of the bending section 109 and connected to a distal end side of the operation section 103.

Note that the endoscope 101 may be an endoscope of a form called rigid endoscope not including a part having flexibility in the insertion section 102.

As explained in detail below, the image pickup unit 1 is provided at the distal end portion 110. An angle operation knob 106 for operating bending of the bending section 109 is provided in the operation section 103.

An endoscope connector 105 connected to an external apparatus 120 is provided at a proximal end portion of the universal cord 104. The external apparatus 120, to which the endoscope connector 105 is connected, is connected to an image display section 121 such as a monitor via a cable.

The endoscope 101 includes a universal cord 104, a composite cable 115 inserted through the operation section 103 and the insertion section 102, and an optical fiber bundle (not shown in the figure), which is a light guide that transmits illumination light emitted from a light source section provided in the external apparatus 120.

The composite cable 115 is configured to electrically connect the endoscope connector 105 and the image pickup unit 1. The endoscope connector 105 is connected to the external apparatus 120, whereby the image pickup unit 1 is electrically connected to the external apparatus 120 via the composite cable 115.

Supply of electric power from the external apparatus 120 to the image pickup unit 1 and communication between the external apparatus 120 and the image pickup unit 1 are performed via the composite cable 115.

An image processing section is provided in the external apparatus 120. The image processing section generates a video signal on the basis of an image pickup device output signal outputted from the image pickup unit 1 and outputs the video signal to the image display section 121. That is, in the present embodiment, an optical image (an endoscopic image) picked up by the image pickup unit 1 is displayed on the image display section 121 as a video.

Note that the endoscope 101 is not limited to the configuration in which the endoscope 101 is connected to the external apparatus 120 or the image display section 121 and may be, for example, a configuration including a part or all of the image processing section or the monitor.

The light guide is configured to transmit light emitted from the light source section of the external apparatus 120 to an illumination window functioning as an illumination-light emitting section of the distal end portion 110. Further, the light source section may be configured to be disposed in the operation section 103 or the distal end portion 110 of the endoscope 101.

Next, a configuration of the image pickup unit 1 provided in the distal end portion 110 is explained. Note that, in the following explanation, a direction from the image pickup unit 1 toward an object (a left direction in the respective figures) is sometimes referred to as distal end, front, or object side and a direction opposite to the direction is sometimes referred to as proximal end, back, or image side.

As shown in Fig. 2, at the distal end portion 110 of the insertion section 102, an observation window 21, two illumination windows 22 in the figure, and a treatment-instrument-channel opening section 23 are disposed on a distal end face 111 of the distal end portion 110.

At the distal end portion 110, as shown in Fig. 3, a distal-end rigid portion 30, which is a metal block body through which the image pickup unit 1 and a tubular member 24 are inserted and held and fixed, is disposed.

In the tubular member 24, an outward flange 24a is formed to which a distal end of a treatment instrument channel 25 is connected and that positions a distal end position of the treatment instrument channel 25.

The treatment instrument channel 25 is disposed on an inside from the insertion section 102 to the operation section 103. A proximal end of the treatment instrument channel 25 is connected to a treatment instrument insert-through port provided in the operation section 103.

A distal end of an exterior tubular member 26, which covers the image pickup unit 1, the tubular member 24, and a distal end portion of the treatment instrument channel 25, is externally fit in and connected to the distal-end rigid portion 30. A proximal end of the exterior tubular member 26 is fit in and connected to a bending piece (not shown in the figure) at a most distal end provided in the bending section 109.

A distal end of bending rubber 28, which covers the bending section 109, is fixed to the exterior tubular member 26 by a bobbin bonding section 27.

Note that, in the distal end portion 110, as shown in Fig. 4, besides the image pickup unit 1, the treatment instrument channel 25 connected to the tubular member 24 and a plurality of, in the figure, a pair of the light guides 29 described above is disposed.

The plurality of light guides 29 are respectively coated with outer skins 29a. Distal end portions of the light guides 29 are inserted through and fixed in the distal-end rigid portion 30 such that distal end faces of the light guides 29 are opposed to a rear surface of the illumination window 22 shown in Fig. 2.

Other components in the endoscope 101 are the same as the components in the past. Therefore, detailed explanation of the components is omitted.

The image pickup unit 1 functioning as the solid-state image pickup apparatus in the present embodiment is explained in detail below.

As shown in Fig. 5, the image pickup unit 1 is mainly configured to include a lens holder 2, an image pickup device holder 3, an image pickup device 4, and a circuit board 5 in order from the object side in the front.

In the lens holder 2, a group of a plurality of objective lens 31 functioning as an objective optical system in the figure is disposed. An objective lens at a most distal end configures the observation window 21 shown in Fig. 2. The lens holder 2 of the image pickup unit 1 is fit with the image pickup device holder 3. In the image pickup device holder 3, a cemented lens 33 is provided on an inside on a proximal end side. The image pickup device holder 3 is bonded to a cover glass 34, which protects a not-shown light receiving section of the solid-state image pickup device 4, after optical axis adjustment is performed.

The image pickup device 4 is an extremely small rectangular electronic component. In the image pickup device 4, a plurality of elements that output electronic signals corresponding to incident photographing light at predetermined timing are arrayed in a planar light receiving section. For example, a form generally referred to as CCD (charge coupled device), CMOS (complementary metal oxide semiconductor) sensor, or the like or other various forms are applied. A rear surface on a proximal end side of the image pickup device 4 is bonded to the circuit board 5.

The circuit board 5 is configured from, for example, a multilayer board as a rigid board, a base material of which is configured from a laminated board of glass epoxy resin or ceramic. The circuit board 5 is surface-bonded to the rear surface of the image pickup device 4 via a thermosetting adhesive or the like. The circuit board 5 includes a core-wire connecting section 5a of a tabular block on which a plurality of electronic components 35 and 36 are mounted and in which not-shown electronic components are embedded on an inside and an external-conductor connecting section 5b functioning as a projecting section extended to project backward while having a step from a proximal end center portion of the core-wire connecting section 5a.

In the circuit board 5 functioning as the rigid board, a flexible printed board (hereinafter described as FPC) 37 is electrically connected to a lower surface of the core-wire connecting section 5a. An inner lead extended from a distal end side of the FPC 37 is electrically connected to a bump formed on a lower front surface of the image pickup device 4. Consequently, a driving power supply is supplied to the image pickup device 4. Exchange of signals between the image pickup device 4 and the circuit board 5 is performed.

On front and rear surfaces in the back of the core-wire connecting section 5a of the circuit board 5, a plurality of conductor lands 43, to which a core wire 41 a of a power supply line 41 and core wires 42a of a plurality of signal lines 42 are connected by solder, are disposed.

On front and rear surfaces of the external-conductor connecting section 5b of the circuit board 5, at least one conductor land 43 and one or a plurality of external conductor lands 44, to which outer diameter sections of external conductors 42c in a peeled state of outer skins 42d in the plurality of signal lines 42 are connected in an extending direction along a longitudinal direction of the circuit board 5, are disposed.

That is, in the circuit board 5, the core wires 41 a and 42a of the power supply line 41 and the signal lines 42 for exchanging an image pickup signal, a driving signal, and the like extended from the composite cable 115 are electrically connected to the conductor lands 43 by solder. The outer diameter sections of the external conductors 42c of the signal lines 42 are electrically connected to the external conductor lands 44 by solder.

Note that coaxial cables are used as the plurality of signal lines 42. The plurality of signal lines 42 are disposed in the composite cable 115 together with the power supply line 41 in the insertion section 102.

As shown in Fig. 6, the composite cable 115 has a configuration in which an outer skin 115b is coated on a comprehensive shield 115a such as a copper wire. The power supply line 41 has a configuration in which the core wire 41a is coated with an outer skin 41b formed from insulating resin.

The plurality of signal lines 42, which are coaxial lines, have a configuration in which the core wires 42a are inserted through dielectrics 42b made of resin that control impedance, for example, thin conductors that cover outer circumferences of the dielectrics 42b are braded, and the external conductors 42c used as GND are coated with the outer skins 42d formed from insulating resin.

The power supply line 41 and the plurality of signal lines 42 extend from a distal end of the composite cable 115 when being electrically connected to the image pickup unit 1. The outer skins 41b and 42d of the respective signal wires 41 and 42 are peeled off.

In the power supply line 41, as shown in Fig. 7, the core wire 41a is electrically connected to, by solder, at least one of the conductor lands 43 on a surface of the core-wire connecting section 5a in the circuit board 5 of the image pickup unit 1, in the figure, on an upper surface side in view toward the paper surface. At this point, as shown in Fig. 8, the power supply line 41 is connected to the conductor lands 43 with the core wire 41a bared such that the outer skin 41b overlaps the external-conductor connecting section 5b.

In the plurality of signal lines 42, as in the power supply line 41, the respective core wires 42a are electrically connected to, by solder, predetermined conductor lands 43 on front and rear surfaces of the core-wire connecting section 5a in the circuit board 5 of the image pickup unit 1, in the figure, upper and lower surfaces in view toward the paper surface (see Fig. 7).

At this point, as shown in Fig. 5, the plurality of signal lines 42 are peeled off to locate distal end positions of the dielectrics 42b in positions close to a proximal end of the core-wire connecting section 5a not to overlap the core-wire connecting section 5a and the core wires 42a are bared. Consequently, the signal lines 42 can be connected in parallel to the laminated board 5a. Therefore, it is possible to prevent a situation in which the signal lines 42 bend in an up-down direction of the paper surface and an external shape of the image pickup unit increases in size.

The external conductors 42c are cut to locate distal ends slightly on a rear side with respect to distal ends of the dielectrics 42b not to be short-circuited with the core wires 42a. Outer circumferential sections functioning as outer diameter sections are electrically connected to, by solder, predetermined external conductor lands 44 provided on front and rear surfaces of the external-conductor connecting section 5b in a state in which the external conductors 42c covers the dielectrics 42b (see Fig. 8).

Note that the plurality of external conductor lands 44 provided for the respective signal lines may be collected for the same GND In the present embodiment, wide cable lands may be respectively disposed on upper and lower surfaces of the external-conductor connecting section 5b.

As shown in Fig. 9, the circuit board 5 is integrally formed by a base material for stacking the core-wire connecting section 5a and the external-conductor connecting section 5b. One or a plurality of, in the figure, two sheets 5c are further stacked on front and rear surfaces of a portion to be the core-wire connecting section 5a. A step of the external-conductor connecting section 5b is formed.

More specifically, first, a dimension difference δt between outer diameter sections of the core wires 42a of the signal lines 42 and outer diameter sections of the external conductors, that is, a sum of thickness of the dielectrics of the signal lines 42 and thickness of the external conductors is thickness of approximately an integral multiple times as large as thickness of the sheet 5c. Note that, in the circuit board 5, in the figure, the dimension difference δt is approximate twice as large as the thickness of the sheet 5c. The two sheets 5c are stacked and a step of the external-conductor connecting section 5b having a predetermined height dimension t1 in an inner diameter direction is formed with respect to the core-wire connecting section 5a.

Therefore, the height dimension t1 of the step of the external-conductor connecting section 5b formed in the circuit board 5 is substantially the same (t1=δt) as the dimension difference δt between the outer diameter sections of the core wires 42a and the outer diameter sections of the external conductors.

Note that a thickness dimension t2 of the conductor lands 43 of the core-wire connecting section 5a and a thickness dimension t3 of the external conductor lands 44 of the external-conductor connecting section 5b are also the same (t2=t3) because the conductor lands 43 and the external conductor lands 44 are the same metal foil.

In this way, in the circuit board 5 to which the signal lines 42 are connected, according to the dimension difference δt between the outer diameter sections of the core wires 42a of the signal lines 42 and the outer diameter sections of the external conductors, one or the plurality of sheets 5c are stacked to form the step of the external-conductor connecting section 5b having the predetermined height dimension t1 in a center direction with respect to the core-wire connecting section 5a.

As explained above, the image pickup unit 1 has a configuration including the core-wire connecting section 5a in which the core wires 41a and 42a of the power supply line 41 and the plurality of signal lines 42 are connected to the circuit board 5 and the external-conductor connecting section 5b that has the step in the external form center direction with respect to the core-wire connecting section 5a and to which the external conductors 42c of the plurality of signal lines 42 are connected.

In the image pickup unit 1, the conductor lands 43 of the core-wire connecting section 5a, to which one signal line 42 is connected, and the external conductor lands 44 of the external-conductor connecting section 5b are disposed in a direction along the longitudinal direction of the circuit board 5.

Consequently, in the image pickup unit 1, the plurality of signal lines 42 can be linearly connected to the circuit board 5 along the longitudinal direction. That is, in the plurality of signal lines 42, the external conductors 42c can be connected to the external conductor lands 44 of the external-conductor connecting section 5b in substantially linear state from a state in which the core wires 42a are connected to the conductor lands 43 of the core-wire connecting section 5a.

In the image pickup unit 1 configured as explained above, the external conductors 42c of the plurality of signal lines 42 do not have to be bound with a jumper wire for ground or the like as in the past. The signal lines 42 can be electrically connected to the external conductor lands 44 for GND in a state in which the outer skins 42d are simply peeled. It is possible to reduce the circuit board 5 in length. It is possible to reduce the circuit board 5 in a diameter because a bound section of the external conductors 42c is absent and the external conductors 42c do not bulge in the outer diameter direction.

That is, in the external-conductor connecting section 5b extending from the circuit board 5, which is the rigid board, to the proximal end side, a space for drawing around the respective signal lines 42 to predetermined positions of the laminated board 5a is used. Since a dead space is effectively used, the rigid length of the circuit board 5 does not increase. It is possible to reduce the circuit board 5 in length.

Consequently, the image pickup unit 1 in the present embodiment can be reduced in size in the configuration in which the image pickup unit 1 is incorporated in the distal end portion 110 of the insertion section 102 of the endoscope 101. Therefore, the distal end portion 110 also decreases in size. It is possible to adopt a configuration contributing to a reduction in a diameter of the insertion section 102.

Further, in the image pickup unit 1, since the step is provided in the circuit board 5, a solder material in soldering the core wires 41 a and 42a of the power supply line 41 and the plurality of signal lines 42 to the conductor lands 43 is prevented from easily flowing to the external-conductor connecting section 5b side by surface tension. It is also possible to prevent a short circuit between the plurality of signal lines 42 and the external conductors 42c. Since it is unnecessary to secure a space in the insertion section longitudinal direction as in the past, there is also an advantage that the rigid length is reduced.

### (Modifications)

The image pickup unit 1 may adopt configurations of various modifications explained below. Note that, in the various modifications explained below, naturally, respective components can be combined.

### (First modification)

Fig. 10 is a sectional view showing a configuration of an external-conductor connecting section of a circuit board of an image pickup unit in a first modification.

As shown in Fig. 10, in the image pickup unit 1, recessed sections 5d having an arcuate shape in section conformed to a shape of the external conductors 42c may be formed in portions where the external conductor lands 44, to which the external conductors 42c of the plurality of signal lines 42 are connected, are provided in the external-conductor connecting section 5b of the circuit board 5.

In this way, in the image pickup unit 1, the recessed sections 5d are formed in the external-conductor connecting section 5b of the circuit board 5 and the external conductor lands 44 are provided on surfaces of the recessed sections 5d. Consequently, it is easy to perform positioning for connecting the signal lines 42. It is easy to solder the external conductors 42c to the external conductor lands 44 and assemblability is improved.

Note that, in the circuit board 5 in this modification, a recessed section 5e having an arcuate shape in section, in which the power supply line 41 is engaged, is also formed in the external-conductor connecting section 5b. Therefore, it is also easy to position the power supply line 41 on the circuit board 5.

Further, a depth dimension of the recessed sections 5d is set to a total (a sum) of respective thickness dimensions of the external conductor lands 44, and the dielectrics 42b and the external conductors 42c of the signal lines 42. Consequently, the step of the external-conductor connecting section 5b does not have to be provided with respect to the core-wire connecting section 5a.

### (Second modification)

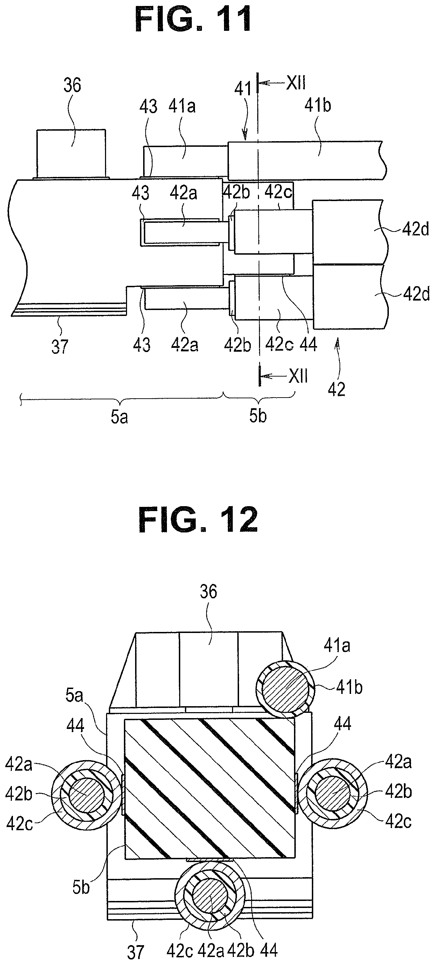

Fig. 11 is a side view showing a configuration of a circuit board to which a power supply line and a plurality of signal lines are connected in a second modification. Fig. 12 is a sectional view showing a configuration of an image pickup unit taken along line XII-XII in Fig. 11.

As shown in Fig. 11 and Fig. 12, the image pickup unit 1 may have a configuration in which the plurality of signal lines 42 are connected to both side surfaces of the external-conductor connecting section 5b of the circuit board 5.

More specifically, in the image pickup unit 1, the conductor lands 43 or the external conductor lands 44, to which the core wires 42a or the external conductors 42c of the signal lines 42 are connected, are formed on both side surfaces of the core-wire connecting section 5a or the external-conductor connecting section 5b of the circuit board 5.

The external-conductor connecting section 5b in this modification has a configuration having a step in a side surface direction as well with respect to the core-wire connecting section 5a. It is possible to linearly connect, along the longitudinal direction, the signal lines 42 connected to both side surfaces of the circuit board 5.

In the image pickup unit 1 having such a configuration, in addition to the effects explained above, it is possible to improve a degree of freedom of design of the circuit board 5.

### (Third modification)

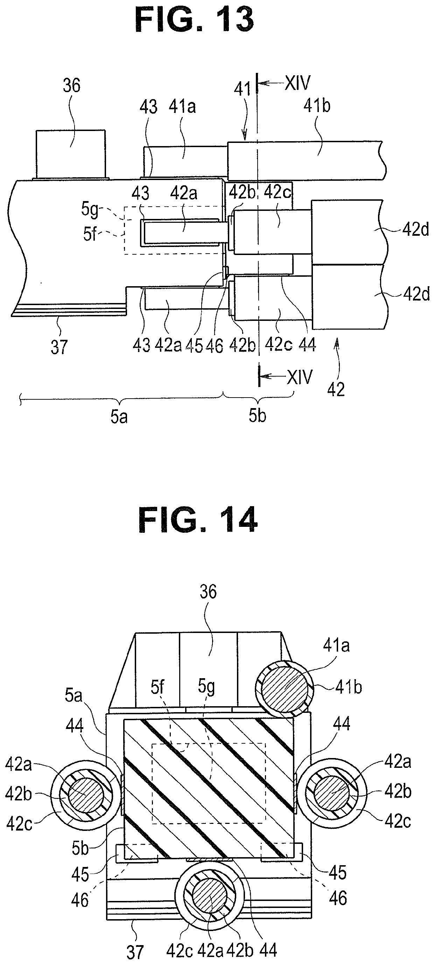

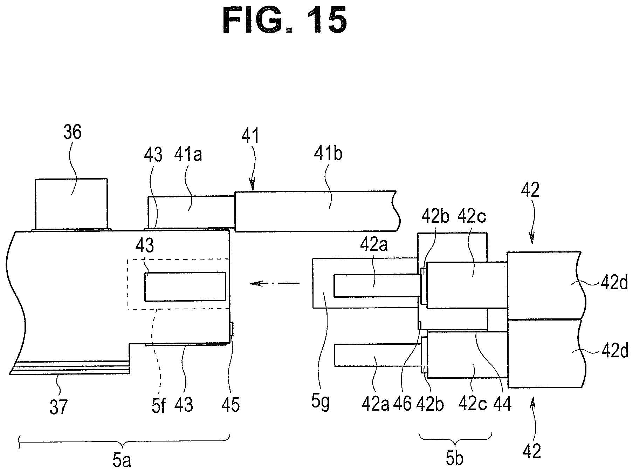

Fig. 13 is a side view showing a configuration of a circuit board to which a power supply line and a plurality of signal lines are connected in a third modification. Fig. 14 is a sectional view showing a configuration of an image pickup unit taken along line XIV-XIV in Fig. 13. Fig. 15 is a side view showing a state before an external-conductor connecting section is bonded to a core-wire connecting section.

As shown in Fig. 13 to Fig. 15, as in the second modification, the image pickup unit 1 in this modification has a configuration in which the plurality of signal lines 42 are connected to both the side surfaces of the external-conductor connecting section 5b of the circuit board 5. The image pickup unit 1 has a separated configuration in which the external-conductor connecting section 5b is detachably attachable to the core-wire connecting section 5a of the circuit board 5.

More specifically, in the circuit board 5, a recessed section 5f having a rectangular shape is formed from a center portion of a proximal end face to an inside in the core-wire connecting section 5a. A projecting section 5g having a rectangular shape similar to the recessed section 5f of the core-wire connecting section 5a is formed from a center portion of a distal end face in the external-conductor connecting section 5b.

In this way, in the circuit board 5, the projecting section 5g of the external-conductor connecting section 5b is engaged with the recessed section 5f of the core-wire connecting section 5a. Consequently, the external-conductor connecting section 5b is attached to the core-wire connecting section 5a. Note that the external-conductor connecting section 5b and the core-wire connecting section 5a are firmly attached by an adhesive or the like.

In the core-wire connecting section 5a, two electric contacts 45 are provided in both side portions on a lower side of a proximal end face. In the external-conductor connecting section 5b, two electric contacts 46, which come into contact with and are electrically connected to the two electric contacts 45 of the core-wire connecting section 5a, are provided in both side portions on a lower side of a proximal end face of a distal end face.

The electric connection of the electric contacts 45 and 46 in contact with each other is secured by fillet-like solder or the like.

Note that the electric contacts 45 and 46 are electrically connected to a conductor for GND In the external-conductor connecting section 5b, the electric contacts 45 and 46 are electrically connected to the external conductor lands 44 via internal wires.

By adopting such a configuration, in the image pickup unit 1, in addition to the effects explained above, in the circuit board 5, after a unit in which the plurality of signal lines 42 are connected to the external-conductor connecting section 5b in advance is assembled, it is possible to attach and fix the external-conductor connecting section 5b to the core-wire connecting section 5a. It is possible to improve assemblability.

Further, in the circuit board 5, since the core-wire connecting section 5a and the external-conductor connecting section 5b are separated, compared with a configuration in which the core-wire connecting section 5a and the external-conductor connecting section 5b are integrated, it is unnecessary to form the small external-conductor connecting section 5b from a block-like stacked body by dicing or the like. It is easy to manufacture the circuit board 5.

### (Fourth modification)

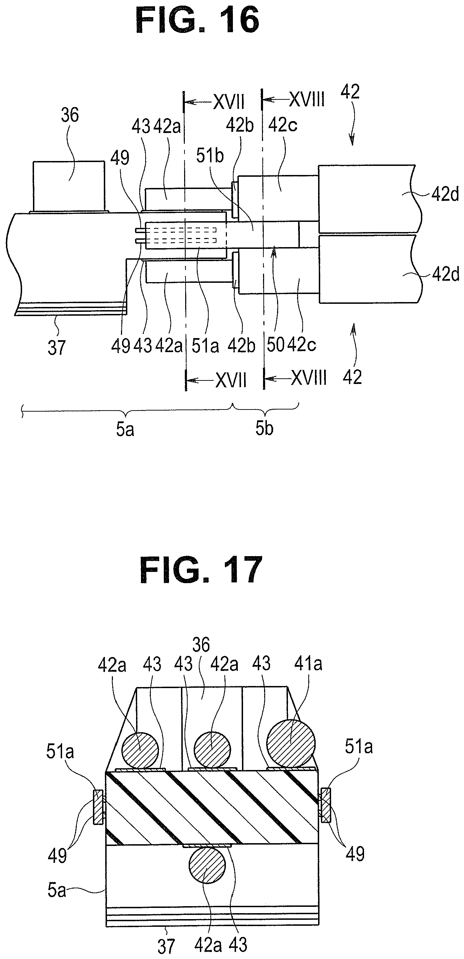

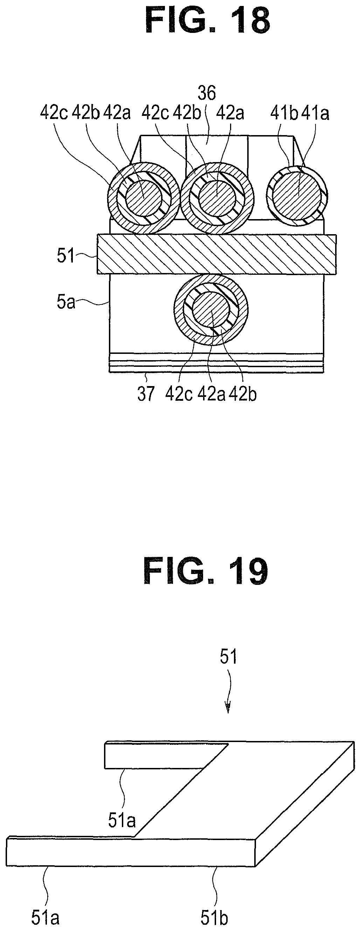

Fig. 16 is a side view showing a configuration of a circuit board to which a plurality of signal lines are connected in a fourth modification. Fig. 17 is a sectional view showing a configuration of an image pickup unit taken along line XVII-XVII in Fig. 16. Fig. 18 is a sectional view showing the configuration of the image pickup unit taken along line XVIII-XVIII in Fig. 16. Fig. 19 is a perspective view showing a configuration of an external-conductor connecting section made of metal.

As shown in Fig. 16 to Fig. 19, the image pickup unit 1 in this modification has a separated configuration in which an external-conductor connecting section 51 having a substantially tabular shape formed from a conductor such as metal is detachably attachable to the core-wire connecting section 5a of the circuit board 5.

More specifically, in the circuit board 5, two electric contacts 49 on one side in the figures is provided on both the side surfaces of the core-wire connecting section 5a. As shown in Fig. 17, two arm sections 51a (see Fig. 19) of the external-conductor connecting section 51 are electrically connected to the electric contacts 49 by solder.

The electric contacts 49 provided in the core-wire connecting section 5a are electrically connected to a conductor for GND by internal wires.

In the plurality of signal lines 42, as shown in Fig. 18, the external conductors 42c are directly electrically connected to front and rear surfaces of a tabular section 51b of the external-conductor connecting section 51 by solder.

Note that, as shown in Fig. 19, the external-conductor connecting section 51 has a configuration in which two arm sections 51a are extended forward from both side sections on a distal end side of the tabular section 51b.

By adopting such a configuration, in the image pickup unit 1, in addition to the effects explained above, as in the third embodiment, in the circuit board 5, after a unit in which the plurality of signal lines 42 are connected to the external-conductor connecting section 51 in advance is assembled, it is possible to attach and fix the external-conductor connecting section 5b to the core-wire connecting section 5a. It is possible to improve assemblability.

Note that, when the external-conductor connecting section 51 is attached and fixed to a desired position of the core-wire connecting section 5a, as explained with reference to Fig. 9, a surface of the core-wire connecting section 5a to which the core wires 42a are connected and a surface of the external-conductor connecting section 5b to which the external conductors 42c are connected are present at determined height.

Further, in the image pickup unit 1, since the external-conductor connecting section 51 itself of the circuit board 5 is a conductor, it is unnecessary to provide external conductor lands for electrically connecting the external conductors 42c of the signal lines 42. It is possible to easily perform electric connection to the core-wire connecting section 5a.

### (Fifth modification)

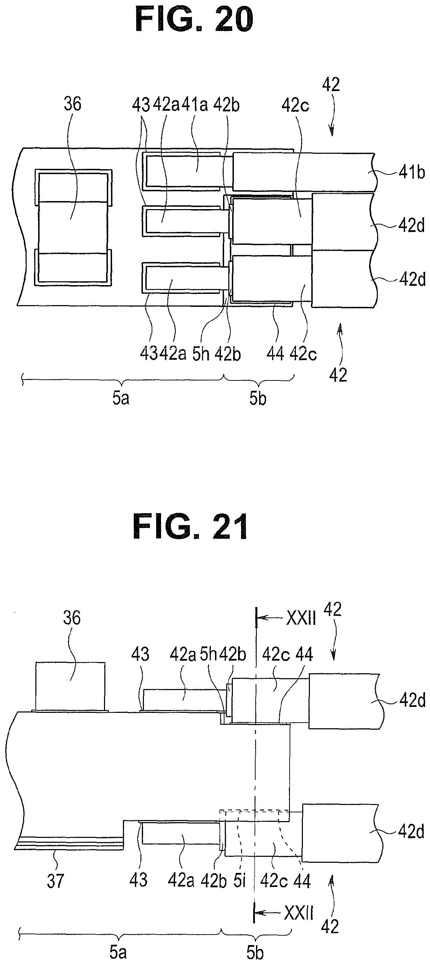

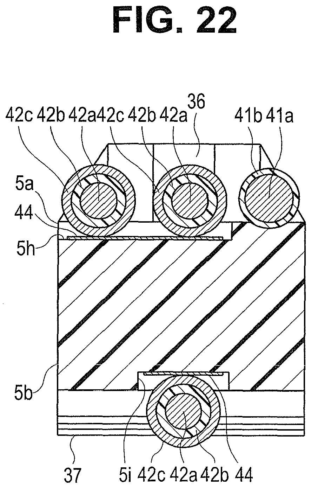

Fig. 20 is a top view showing a configuration of a circuit board to which a plurality of signal lines are connected in a fifth modification. Fig. 21 is a side view showing a configuration of a circuit board to which a plurality of signal lines are connected. Fig. 22 is a sectional view showing a configuration of an image pickup unit taken along line XXII-XXII in Fig. 21.

As shown in Fig. 20 and Fig. 21, the image pickup unit 1 in this modification includes, on front and rear surfaces of the external-conductor connecting section 5b of the circuit board 5, step sections 5h and 5i formed by cutting out or grooving portions where the external conductor lands 44 to which the external conductors 42c of the plurality of signal lines 42 are connected are provided.

Note that the step section 5i formed on the rear surface of the external-conductor connecting section 5b is formed such that a cross section of the external-conductor connecting section 5b is formed in a concave shape.

In this way, in the image pickup unit 1, the step sections 5h and 5i are formed on the front and rear surfaces of the external-conductor connecting section 5b of the circuit board 5 and the external conductor lands 44 are provided on respective surfaces of the step sections 5h and 5i. Consequently, as in the first modification, it is easy to perform positioning for connecting the signal lines 42. It is easy to solder the external conductors 42c to the external conductor lands 44 and assemblability is improved.

Compared with the configuration in which the semicircular recessed sections 5d are formed as in the first modification, the image pickup unit 1 in this modification has a configuration with high machinability because the step sections 5h and 5i can be easily formed on the front and rear surfaces of the external-conductor connecting section 5b.

Further, in this modification, as in the first modification, a depth dimension of the step sections 5h and 5i is set to a total (a sum) of respective thickness dimensions of the external conductor lands 44, and the dielectrics 42b and the external conductors 42c of the signal lines 42. Consequently, a step does not have to be further provided in the external-conductor connecting section 5b with respect to the core-wire connecting section 5a.

Note that, in the image pickup unit 1 in the embodiment and the modifications, the image pickup device 4 of a so-called vertical type is illustrated. However, the present invention is also applicable to a configuration of a so-called horizontal type that detects photographing light refracted using a reflection member such as a prism.

A so-called flexible endoscope is illustrated as the endoscope 101. However, naturally, the present invention is a technique applicable to not only a rigid endoscope used in a surgical operation and the like and electronic endoscopes including only endoscopes for medical use but also all kinds of the image pickup unit 1 such as endoscopes for industrial use.

The invention described in the respective embodiments explained above is not limited to the embodiments and the modifications and, besides, can be variously modified and implemented without departing from the spirit of the invention in an implementation stage. Further, the embodiments include inventions in various stages. Various inventions can be extracted by appropriate combinations in a disclosed plurality of constituent elements.

For example, when the described problems can be solved and the described effects can be obtained even if several constituent elements are deleted from all the constituent elements described in the embodiments, a configuration from which the constituent elements are deleted can be extracted as an invention.

The present application is based upon and claims priority from Japanese Patent Application No. 2014-213605 filed in Japan on October 20, 2014, disclosed contents of which are incorporated in the specification, claims, and drawings of the present application.

## Claims

1. A solid-state image pickup apparatus comprising:
an image pickup device configured to detect an object image;
a rigid board electrically connected to the image pickup device; and
a signal cable incorporating at least one or more coaxial lines, wherein
the rigid board includes:
a first surface including a core-wire connecting section to which core wires of the coaxial lines are connected; and
a second surface including an external-conductor connecting section to which external conductors of the coaxial lines are connected, the second surface being substantially parallel to the first surface, and
the coaxial lines are connected substantially in parallel to the first and second surfaces of the rigid board.

2. The solid-state image pickup apparatus according to claim 1, wherein the second surface including the external-conductor connecting section includes a step in an external form center direction with respect to the first surface including the core-wire connecting section.

3. The solid-state image pickup apparatus according to claim 2, wherein a height dimension of the step is substantially same as a dimension difference between an outer diameter section of the core wires of the coaxial lines and an outer diameter section of the external conductors.

4. The solid-state image pickup apparatus according to claim 2 or claim 3, wherein the step in the first surface and the second surface is formed by changing a number of stacked layers of a sheet-like board.

5. The solid-state image pickup apparatus according to claim 4, wherein the dimension difference between the outer diameter section of the core wires of the coaxial lines and the outer diameter section of the external conductors is approximately an integral multiple times as large as the sheet-like board.

6. The solid-state image pickup apparatus according to any one of claims 1 to 5, wherein the coaxial lines are connected to front and rear surfaces or side surfaces of the rigid board.

7. The solid-state image pickup apparatus according to any one of claims 1 to 6, wherein the solid-state image pickup apparatus includes the external-conductor connecting section detachably attachable to the core-wire connecting section.

8. The solid-state image pickup apparatus according to any one of claims 1 to 7, wherein at least one or more external conductor lands, to which outer circumferential sections of the external conductors of the coaxial lines are electrically connected, are disposed in the core-wire connecting section.

9. The solid-state image pickup apparatus according to any one of claims 1 to 7, wherein the external-conductor connecting section is formed of a conductor.

10. An electronic endoscope comprising:
the solid-state image pickup apparatus according to any one of claims 1 to 9; and
an insertion section, in a distal end portion of which the image pickup unit is incorporated, wherein
the signal cable is disposed substantially in parallel to a longitudinal direction of the insertion section.
